# EUROPEAN PATENT APPLICATION

(11) **EP 2 609 929 A1**
(43) Date of publication of application: **03.07.2013**
(21) Application number: 10856466.7
(22) Date of filing: 27.08.2010
(51) Int. Cl.: A61K 36/896, A61K 31/4375, A61P 25/28, A61P 25/00

(54) **COMPOSITION FOR PREVENTING OR TREATING DEMENTIA**

(71) Applicant: Korea Institute of Science and Technology, Seoul 136-791 (KR); Huazhong University of Science and Technology, Wuhan City Hubei 430074 (CN)
(72) Inventor: YANG, Hyun-Ok, Seoul 135-774 (KR); KWON, Hak-Cheol, Seoul 135-240 (KR); PARK, Jin-Soo, Gangneung-si Gangwon-do 210-101 (KR); CHUNG, Sung-Kwon, Seoul 138-220 (KR); WU, ZhiZou, Hubei 430030 (CN); RUAN, Hanli, Hubei 430030 (CN); PI, Huifang, Hubei 430030 (CN); ZHANG, Peng, Hubei 430030 (CN)
(74) Representative: advotec.
(86) International application number: PCT/KR2010/005796
(87) International publication number: WO 2012/026641

(57) **Abstract**

There is provided use of Lycoris aurea extracts, and/or fractions thereof, and/or lycoricidine and lycoricidinol separated from the extracts or fractions for prevention and/or treatment of dementia.

## Description

### FIELD OF THE INVENTION

The present invention relates to use of Lycoris aurea extracts and/or fractions thereof, and/or lycoricidine and lycoricidinol compounds separated from the extracts or fractions for prevention and/or treatment of dementia.

### BACKGROUND OF THE INVENTION

As the world is slowly moving toward an aging society, there is a sharp increase of the people who suffer from various kinds of brain diseases such as a stroke, Parkinson's disease and Alzheimer's disease, and the cost is astronomical. Alzheimer's disease, which is generated by temporary or persistent damage of brain nerves, is a progressive and degenerative disease characterized by the manifestation of the overall disorder of mental functions. Although the cause of the disease hasn't been clearly elucidated so far, it was known to be closely related to aging. It was known that during the process of aging, the brain is deposited with a protein called beta-amyloid, which produces a tangled plaque, and causes the Alzheimer's disease (Breimer LH, et al. Nature, 326: 749-750, 1987). In the past, the studies for treatment of dementia diseases were conducted using an enzyme called acetylcholine esterase as a primary site of drug action, but it has been known that the inhibition of the activity of this enzyme only leads a normal life by inhibiting the decline phenomena of the neurotransmitter, acetylcholine, which turns up in dementia patients due to Alzheimer's disease, and it does not eliminate a root cause of the disease.

Those developed so far and approved by FDA as medicines for Alzheimer's disease are acethyl cholinesterase inhibitors (AchEIs) and N-methyl D-aspartate receptor antagonists (NMDARA) type compounds and currently, about 15 medicines are under clinic tests led by multinational pharmaceutical companies. However, since such medicines are not able to become a root medicine for Alzheimer's disease, there is a need for the development of beta or gamma-secretase suppressor and beta-amyloid deposition suppressors to reduce the production of beta-amyloids which are assumed to be a main cause of Alzheimer's disease.

### SUMMARY OF THE INVENTION

In accordance with one embodiment of the present invention, there is provided a composition for inhibition of beta-amyloid production, containing the extract of Lycoris aurea as an active ingredient.

In accordance with another embodiment, there is provided a composition and health functional food for prevention and/or treatment of dementia, containing the extract of Lycoris aurea as an active ingredient.

In accordance with another embodiment, there is provided a composition for inhibition of beta-amyloid production, containing at least one selected from the group consisting of lycoricidine, lycoricidinol, and a pharmaceutically acceptable salt thereof as an active ingredient.

In accordance with another embodiment, there is provided a composition and health functional food for prevention and/or treatment of dementia, containing at least one selected from the group consisting of lycoricidine, lycoricidinol, and a pharmaceutically acceptable salt thereof as an active ingredient.

In accordance with another embodiment, there is provided a use of the extract of Lycoris aurea for inhibition of beta-amyloid production, or for preparation of a beta-amyloid production inhibitor.

In accordance with another embodiment, there is provided a use of the extract of Lycoris aurea for prevention and/or treatment of dementia, or for preparation of a preventive agent and/or treatment agent for dementia.

In accordance with another embodiment, there is provided a use of at least one selected from the group consisting of lycoricidine, lycoricidinol, and a pharmaceutically acceptable salt thereof, for inhibition of beta-amyloid production, or for preparation of a beta-amyloid production inhibitor.

In accordance with another embodiment, there is provided a use of at least one selected from the group consisting of lycoricidine, lycoricidinol, and a pharmaceutically acceptable salt thereof, for prevention and/or treatment of dementia, or for preparation of a preventive agent and/or treatment agent for dementia.

In accordance with another embodiment, there is provided a method for inhibition of beta-amyloid production, comprising identifying a patient who is in need of the inhibition of beta-amyloid production and administering the extract of Lycoris aurea to the patient.

In accordance with another embodiment, there is provided a method for prevention and/or treatment of dementia, comprising identifying a patient who is in need of prevention and/or treatment of dementia and administering the extract of Lycoris aurea to the patient.

In accordance with another embodiment, there is provided a method for inhibition of beta-amyloid production, comprising identifying a patient who is in need of the inhibition of beta-amyloid production and administering to the patient at least one selected from the group consisting of lycoricidine, lycoricidinol, and a pharmaceutically acceptable salt thereof.

In accordance with another embodiment, there is provided a method for prevention and/or treatment of dementia, comprising identifying a patient who is in need of the prevention and/or treatment of dementia and administering to the patient at least one selected from the group consisting of lycoricidine, lycoricidinol, and a pharmaceutically acceptable salt thereof.

In accordance with another embodiment, there is provided a method for preparation of Lycoris aurea extract having excellent effects on the inhibition of beta-amyloid production, and/or the prevention and/or treatment of dementia.

### DETAILED DESCRIPTION OF THE INVENTION

The inventors have identified that Lycoris aurea extracts, and/or fractions thereof, and/or compounds separated from the extracts or the fractions, lycoricidinol and lycoricidine could inhibit beta-amyloid production through in vitro experiments, thereby to have prevention and/or treatment effects of dementia, particularly Alzheimer's disease, and thus completed the invention.

Beta-amyloid (A*β*) is produced from an amyloid precursor protein (APP) by successive action of beta-secretase (BACE1) and gamma-secretase which are membrane proteases. First, sAPP*β* is produced by the action of beta-secretase and then, the beta-amyloid is produced by the action of gamma-secretase thereon. The beta-amyloid comprises A*β*40 and A*β*42 each of which consists of 40 and 42 amino acids. A*β*40 takes up most of it, but A*β*42, which is produced in a relatively smaller amount, is blamed for the most important cause substance of the diseases because it can readily form a plaque. APP can be hydrolyzed in another route which is not affected by the action of beta-secretase and in this case, sAPPα is first produced by alpha-secretase and then it undergoes the successive action of gamma-secretase to produce a harmless substance, p3. That is, no beta-amyloid is produced in this process. Accordingly, it was reported that if a mouse where BACE1 (beta-site amyloid precursor protein (APP)-cleaving enzyme) is genetically eliminated is created, beta-amyloid deposition does not occur, and cognitive disorder expressed in mice with Alzheimer's disease is inhibited (Laird et al. J. Neuroscience 25: 11693-11709, 2005; Ohno et al. Neuron 41: 27-33, 2004). These results show that the beta-secretase is an important enzyme for the formation of beta-amyloid, suggesting the possibility that its suppressor can be applied as a medicine for dementia, in particular, Alzheimer's disease. In addition, a substance capable of changing the activity of alpha-secretase is expected to be an important target for a treatment of Alzheimer's disease. Furthermore, as the ultimate enzyme of producing the beta-amyloid is the gamma-secretase, an effective suppressor against it is expected to be applicable as a medicine.

Lycoris aurea is a whole herb of Lycoris plant in the Amaryllidaceae family. Lycoris aurea is distributed throughout Korea (Jeju, Jun-nam, and Choong-nam), Japan, Taiwan, and China, and it has been known to contain alkaloid and flavonoid components. Although the anticancer effects of the Lycoris aurea extracts were known (Nerome, K. et al. W02008078362A1), an inhibition of beta-amyloid production or any effects thereof associated with the prevention and treatment of dementia (e.g., Alzheimer's disease) have not reported so far.

Lycoricidine and lycoricidinol separated as an active ingredient for the inhibition of beta-amyloid production in the present invention are known to exist in the plants of genera Narcissus, Lycoris, Pancreatium, and Haemanthus. The lycoricidine and lycoricidinol are known to have anti-viral effects (Gabrielsen, B. et al. J. Nat. Prod. 55: 1569, 1992) and anti-cancer effects (Mondon, A. et al. Chem. Ber. 108: 445, 1975), but up to now, there is no report about the inhibition of beta-amyloid production or effects thereof associated with the prevention and treatment of dementia (Alzheimer's disease).

Hereafter, the invention will be described in more detail.

First, one embodiment of the present invention provides a use of Lycoris aurea extract for inhibition of beta-amyloid production, or for prevention and/or improvement and/or alleviation and/or treatment of dementia.

Therefore, one embodiment of the invention is directed to an inhibitor of beta-amyloid production and/or a composition for prevention and/or treatment of dementia, containing Lycoris aurea extract as an active ingredient. Another embodiment of the invention is directed to a novel use of Lycoris aurea extract for the preparation of an inhibitor of beta-amyloid production and/or a composition for prevention and/or treatment of dementia. Still another embodiment of the invention is directed to a method for inhibition of beta-amyloid production characterized by administering Lycoris aurea extract to a patient who is in need of the inhibition of beta-amyloid production or a method for prevention and/or treatment of dementia, characterized by administering Lycoris aurea extract to a patient who is in need of the prevention and/or treatment of dementia. These methods may further comprise, prior to the administration step, a step of identifying a patient who is in need of the inhibition of beta-amyloid production or a step of identifying a patient who is in need of the prevention and/or treatment of dementia. The patient may be mammals, preferably, human beings.

The dementia may include Alzheimer's disease and vascular dementia and in a particularly preferred example, the dementia may be Alzheimer's disease.

In order to increase the content of an active ingredient which is effective for the suppression of beta-amyloid production, the Lycoris aurea extract may be those obtained by extracting Lycoris aurea with one or more solvents selected from the group consisting of water and a short-chain alcohol of C1 to C4, preferably methanol, ethanol, or isopropanol of 50 to 95 v/v%.

In order to increase the content of an active ingredient which is effective for the suppression of beta-amyloid production, the Lycoris aurea extract may be an ethyl acetate fraction, a butanol fraction or a mixture thereof of the solvent fractions obtained by extracting Lycoris aurea with one or more solvents selected from the group consisting of water and a short-chain alcohol of C1 to C4, preferably methanol, ethanol, or isopropanol of 50 to 95 v/v% and then fractioning the obtained extract with hexane, methylene chloride, ethyl acetate and butanol in sequence.

In a more preferred embodiment, the Lycoris aurea extract may be a subfraction obtained by performing a reversed-phase chromatography for a mixture of the ethyl acetate fraction and the butanol fraction obtained in the above, using a mixture solution of acetonitrile and water as an eluent, and it may be those having excellent suppressive effects against beta-amyloid production, by containing high amounts of active ingredients, lycoricidine and/or lycoricidinol as described below. Of the mixture solution of acetonitrile and water used as the eluent, the content of acetonitrile may be preferably 10 to 50 v/v% and in particular, in case of 31 to 33 v/v%, a subfraction with a high amount of lycoricidine can be obtained and in case of 42 to 44 v/v%, a subfraction with a high amount of lycoricidinol can be obtained. The content of lycoricidine in the subfraction in the case of 31 to 33 v/v% may be not less than about 80 w/w%, for example, 80 to 99.9 w/w%, preferably 90 to 99.9 w/w%. Such content of lycoricidine corresponds to about 0.04 to 0.1 w/w% or so on the basis of the weight of the crude extract extracted with the initial mixture of water and the short-chain alcohol of C1-C4. The content of lycoricidinol in the subfraction in the case of 42 to 44 v/v% may be not less than about 80 w/w%, for example, 80 to 99.9 w/w%, preferably 90 to 99.9 w/w%. Such content of lycoricidinol corresponds to about 0.02 to 0.05 w/w% or so on the basis of the weight of the crude extract extracted with the initial mixture of water and the short-chain alcohol of C1-C4.

In another embodiment, the inventors analyzed the active ingredients of the Lycoris aurea extracts and/or fractions and/or subfractions and as a result, they found that the compounds, lycoricidine represented by chemical formula 1 below and lycoricidinol represented by chemical formula 2 below are effective for inhibition of beta-amyloid production and/or for inhibition of alpha- or beta-secretase activity.

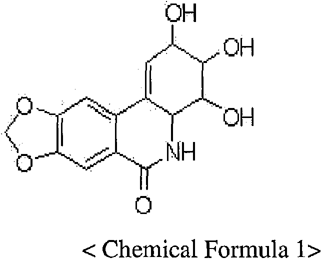

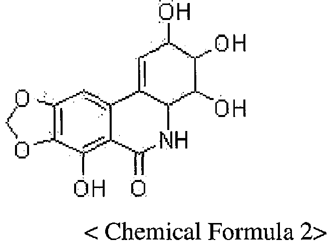

Therefore, another embodiment of the invention is directed to an inhibitor of beta-amyloid production and/or a composition for prevention and/or treatment of dementia, containing at least one selected from the group consisting of lycoricidine represented by chemical formula 1, lycoricidinol represented by chemical formula 2, and a pharmaceutically acceptable salt thereof as an active ingredient. Still another embodiment of the invention is directed to a novel use of at least one selected from the group consisting of lycoricidine represented by chemical formula 1, lycoricidinol represented by chemical formula 2, and a pharmaceutically acceptable salt thereof for the preparation of an inhibitor of beta-amyloid production and/or a composition for prevention and/or treatment of dementia. Still another embodiment of the invention is directed to a method for inhibition of beta-amyloid production characterized by administering at least one selected from the group consisting of lycoricidine represented by chemical formula 1, lycoricidinol represented by chemical formula 2, and a pharmaceutically acceptable salt thereof to a patient who is in need of the inhibition of beta-amyloid production or a method for prevention and/or treatment of dementia, characterized by administering at least one selected from the group consisting of lycoricidine represented by chemical formula 1, lycoricidinol represented by chemical formula 2, and a pharmaceutically acceptable salt thereof to a patient who is in need of the prevention and/or treatment of dementia. These methods may further comprise, prior to the administration step, a step of identifying a patient who is in need of the inhibition of beta-amyloid production or a step of identifying a patient who is in need of the prevention and/or treatment of dementia. The patient may be mammals, preferably, human beings.

The dementia may include Alzheimer's disease and vascular dementia and in a particularly preferred example, the dementia may be Alzheimer's disease.

The lycoricidine represented by formula 1 above is a light yellow semi-solid substance of which the molecular formula is C₁₄H₁₃NO₆ and the molecular weight is 291. The lycoricidinol represented by formula 2 is a light yellow semi-solid substance of which the molecular formula is C₁₄H₁₃NO₇ and the molecular weight is 307. The lycoricidine and the lycoricidinol exhibit very excellent in vitro inhibitory activity against beta-amyloid production so they can be usefully used for prevention and treatment of dementia, particularly Alzheimer's disease. Moreover, the lycoricidine and the lycoricidinol can be applied as a positive control not only in a clinic but also in an animal model and in vitro model, as an inhibitor of beta-amyloid production as a monotherapy.

With regard to the suppression of beta-amyloid production and/or the prevention and/or treatment of dementia, particularly Alzheimer's disease, as the Lycoris extracts, the fractions prepared therefrom (or subfractions), lycoricidine and lycoricidinol are ingredients of herb medicines or ingredients extracted from herb medicines, they have an advantage of minimizing or avoiding any side effects caused by the existing dementia (e.g., Alzheimer's disease) medicines and they may be applied as a monotherapy by the sole administration of one or more kinds of the above ingredients, or they may be applied as a combination therapy by a combined administration along with the existing medicines, Tacrine, Donepezil, Rivastigmine, etc.

Another embodiment of the invention is directed to a health functional food for prevention and/or treatment of dementia, comprising the composition for prevention and/or treatment of dementia. The dementia may include Alzheimer's disease and vascular dementia and in a particularly preferred example, the dementia is Alzheimer's disease.

The health functional food may include all kinds of various foods and/or drinks for prevention and/or improvement of dementia and for example, it may be various foods, drinks, gum, tea, vitamin complex, health functional food supplements, food additives and may be in the form of powders, granules, a tablet, a capsule or a drink. The content of the composition for the prevention and/or treatment of dementia in the food functional foods may be suitably adjusted in light of the purpose, use, object, form, etc. of the final product and for example, the Lycoris aurea extracts, the fractions prepared therefrom (or the subfractions), lycoricidine and/or lycoricidinol may be 0.00001 to 50 wt%, preferably, 0.0001 to 10 wt% on the basis of the solids content, but not limited thereto.

Still another embodiment of the invention is directed to a method for preparation of Lycoris aurea extracts (including fractions and/or sub fractions) having excellent suppressive activity against beta-amyloid. The preparation method may comprise the following steps:
a step of extracting Lycoris aurea with one or more solvents selected from the group consisting of water and a short-chain alcohol of C1 to C4 (step 1);
a step of applying hexane, methylene chloride, ethyl acetate, and butanol in sequence to the extract prepared in step 1 to obtain individual solvent fractions (step 2); and
a step of performing a reversed-phase column chromatography for an ethyl acetate fraction and a butanol fraction of the fractions obtained in step 2 using a mixture solution of acetonitrile and water as an eluent to obtain a subfraction.

The thus obtained subfractions are characterized by containing lycoricidine and lycoricidinol as a main ingredient.

The step 1 is to extract Lycoris aurea with one or more solvents selected from the group consisting of water and a short-chain alcohol of C1 to C4. In a preferred embodiment, the extracting solvent may be methanol, ethanol, or isopropanol of 50 to 95 v/v%. There may be used any Lycoris aurea which is either cultivated or commercially available in the market, and the whole herb or a bulb thereof can be used, that is, there is no restriction on parts to be used, but they are cleanly washed and dried in the shade before their use. There are no special restrictions on the extracting methods, that is, any ordinary methods including heat extraction, ultrasonic extraction, filtration, pressed extraction, reflux extraction, supercritical extraction, or electrical extraction can be applied. In one embodiment, the whole herbs of dried Lycoris aurea are crushed into a suitable size and then placed into an extraction vessel, to which water or a short-chain alcohol of C1 to C4 or a mixture solvent thereof, preferably, methanol, ethanol, or isopropanol of 50 to 95 v/v% are added and then the thus obtained solution is boiled and extracted under reflux and then, let stand for a certain time and filtrated to eliminate residues, thereby to obtain alcohol extracts. The volume of the alcohol solvent may be preferably one to five times the volume of the crushed specimens. The extraction time may be preferably 1 to 12 hours, most preferably 3 hours. Thereafter, it may be further subject to ordinary processes including concentration or lyophilization.

The step 2 is to suspend the extract extracted from step 1 above with water using any ordinarily known methods and then to fraction it with hexane, methylene chloride, ethyl acetate and butanol in sequence, to obtain solvent fractions. For example, about 5 to 100 volume times, preferably 10 to 60 volume times of water on the basis of the weight of the extract are added to the extract obtained in the above, which are then suspended. After that, hexane is added to separate a hexane fraction, the residues are treated with methylene chloride to separate a methylene chloride fraction, the thus remaining residues are treated with ethyl acetate to separate an ethyl acetate fraction, and the thus remaining residues are treated with butanol to separate a butanol fraction. The amount of hexane, methylene chloride, ethyl acetate and butanol to be used may be about 5 to 100 volume times, preferably 10 to 60 volume times on the basis of the weight of the extract, and may be equal to the amount of the water used in the suspension. Of them, the ethyl acetate fraction and the butanol fraction have more excellent inhibitory effects against beta-amyloid so that it is preferable to use a mixture of the ethyl acetate fraction and the butanol fraction in the next step.

The step 3 may be performed by using a mixture solution of acetonitrile and water as an eluent and a reversed-phase liquid chromatography, and it may proceed with the content of acetonitrile being increased. As confirmed in the following test examples, in order to obtain a fraction having excellent inhibitory effects against beta-amyloid production, it is preferable to proceed with the content of acetonitrile being increased, for example, by changing its concentration gradient from 10:90 (acetonitrile:water by volume) to 50:50 (acetonitrile:water by volume), that is, with the concentration of acetonitrile being increased from 10 to 50 v/v%. The mixture solution of acetonitrile and water used as the eluent may include 0.001 to 0.004 v/v%, preferably about 0.002 v/v% of trifluoroacetic acid. In one embodiment of the invention, when eluted using acetonitrile/water in 10 v/v% to 30 v/v% (named 'CN1-M-E-fr1'), 31 v/v% to 33 v/v% (named 'CN1-M-E-fr2'), 42 v/v% to 44 v/v% (named 'CN1-M-E-fr3'), 45 v/v% to 50 v/v% (named 'CN1-M-E-fr4') as eluents, the eluted solutions are dried under reduced pressure to prepare 4 subfractions. Of the fractions, CN1-M-E-fr2 subfraction includes lycoricidine as a main component in an amount of about not less than 80 w/w%, for example, 80 to 99.9 w/w%, preferably 90 to 99.9 w/w%, and CN1-M-E-fr3 subfraction includes lycoricidinol as a main component in an amount of not less than about 80 w/w%, for example, 80 to 99.9 w/w%, preferably 90 to 99.9 w/w%.

In another embodiment of the invention, the preparation method may further comprise a step (step 4) of purifying lycoricidine represented by chemical formula 1 and lycoricidinol represented by chemical formula 2 by re-performing a reversed-phase column chromatography for CN1-M-E-fr2 subfraction having lycoricidine as a main component and CN1-M-E-fr3 subfraction having lycoricidinol as a main component prepared in step 3, respectively. For the purification of lycoricidine, a reversed-phase column chromatography may be performed for the above subfractions, preferably, CN1-M-E-fr2 subfraction having lycoricidine as its main component, using the mixture solution of acetonitrile and water containing 0.05 to 0.2 v/v%, preferably 0.1 v/v% trifluoroacetic acid as an eluent with a concentration gradient of increasing the composition of acetonitrile from 10 v/v% acetonitrile/water to 20 v/v% acetonitrile for 40 min, to purify lycoricidine represented by chemical formula 1. For the purification of lycoricidinol, it may be performed for the above subfractions, preferably, CN1-M-E-fr3 subfraction having lycoricidinol as its main component, using the mixture solution of acetonitrile and water as an eluent with a concentration gradient of increasing the composition of acetonitrile from 10 v/v% acetonitrile/water to 20 v/v% acetonitrile for 20 min, to purify lycoricidinol represented by chemical formula 2.

The Lycoris aurea extracts, fractions, lycoricidine and lycoricidinol of the present invention may be applied as either monotherapy or combination therapy along with the existing dementia (e.g., Alzheimer's disease) medicines such as Tacrine, Donepezil, and Rivastigmine, or they may be applied as a herb medicine composition so that they can be usefully used for the prevention and/or treatment of dementia, in particular, Alzheimer's disease.

### BRIEF DESCRIPTION OF THE DRAWINGS

Fig. 1 shows inhibitory effects against A*β*42 and A*β*40 production according to the concentrations of Lycoris aurea alcohol extracts (CN1-M).
Fig. 2 shows inhibitory effects against A*β*42 production of 4 kinds of solvent fractions (CN1-MH, CN1-MM, CN1-ME, and CN1-MB) prepared from the Lycoris aurea alcohol extracts at a concentration of 25 *µ*g/ml.
Fig. 3 shows inhibitory effects against A*β*42 production of 4 kinds of subfractions (CN1-M-E-fr1, CN1-M-E-fr2, CN1-M-E-fr3, and CN1-M-E-fr4) prepared from the combined fractions (CN1-M-E) of the ethyl acetate fraction (CN1-ME) and the butanol fraction (CN1-MB) of the Lycoris aurea alcohol extracts at a concentration of 25 *µ*g/ml.
Fig. 4 shows inhibitory effects against A*β*42 production according to the concentrations of the compounds lycoricidine (CN1-M-E2) and lycoricidinol (CN1-M-E3).
Fig. 5 shows reduction effects by the compounds lycoricidine (CN1-M-E2) and lycoricidinol (CN1-M-E3) on the production of sAPPα which is resulted from the activity of beta-secretase.
Fig. 6 shows reduction effects of lycoricidine and lycoricidinol on the production of sAPPα and sAPP*β*.
Fig. 7 shows band densities of sAPP*α* and sAPP*β* in Western blot results when treated with lycoricidine and lycoricidinol.
Fig. 8 shows cell death activity according to the concentrations of lycoricidine (CN1-M-E2) and lycoricidinol (CN1-M-E3).

### EXAMPLES

Hereafter, the present invention will be described in more detail by virtue of the following examples. They are intended to merely illustrate the invention and not construed to limit the scope of the invention in any way.

### Example 1: Preparation of Lycoris Aurea Alcohol Extracts

Lycoris aurea which was collected in Wuhan, Hubei, China was used in this example. 200 g of bulbs of Lycoris aurea which were dried in the shade were finely cut and placed into an extracting vessel, to which 95 v/v% ethanol were added in two volume times the specimen volume. Then, it was extracted under reflux for 3 hours, cooled down to a room temperature, and then filtrated. The extracted solution was concentrated under a reduced pressure at 40 °C until the solvent was completely evaporated, to obtain 50 g of alcohol extracts (CN1-M).

### Example 2: Preparation of Lycoris Aurea Solvent Fractions and Subfractions of Ethyl Acetate Fraction

25 g of the Lycoris aurea alcohol extracts prepared in Example 1 above were suspended in 1L of water, to which 1L of hexane, methylene chloride, ethyl acetate, and butanol were added in sequence each two times to fraction solvents (premium level of the solvents are each used) and fractions were thus obtained in amounts of 1.3 g (hexane fraction), 94 mg (methylene chloride fraction), 90 mg (ethyl acetate fraction) and 1.2 g (butanol fraction), and the remaining water fraction was 22 g.

Thereafter, a C18 reversed-phase chromatography was performed for 1.29 g of a combined fraction of the ethyl acetate fraction and the butanol fraction using a mixture solution of acetonitrile and water containing 0.02% trifluoroacetic acid as an eluent, by maintaining 10% acetonitrile/water for 5 min., and then changing the ratio of acetonitrile:water from 10:90 by volume to 50:50 by volume for 60 min., thereby to obtain 4 types of subfractions of CN1-M-E-fr1 from retention time of 8 to 36 min. (acetonitrile content: 10 v/v% to 30 v/v%), CN1-M-E-fr2 from 36 to 53 min. (acetonitrile content: 31 v/v% to 33 v/v%), CN1-M-E-fr3 from 53 to 56 min. (acetonitrile content: 42 v/v% to 44 v/v%) and CN1-M-E-fr4 from 56 to 60 min. (acetonitrile content: 45 v/v% to 50 v/v%). All 4 types of the subfractions contained lycoricidine or lycoricidinol represented by chemical formula 1 or 2 but among them, the CN1-M-E-fr2 subfraction contained about 80% or more of the lycoricidine as a main component and the CN1-M-E-fr3 subfraction contained about 80% or more of the lycoricidinol as a main component.

### Example 3: Purification and Structure Identification of Lycoricidine

The inventors concentrated the subfraction (CN1-M-E-fr2) containing the lycoricidine as a main component prepared in Example 2 under a reduced pressure and then, performed a high performance liquid chromatography separation with a C18 reversed-phase column using a mixture solution of acetonitrile and water containing 0.1 v/v% trifluoroacetic acid as an eluent with a concentration gradient of increasing the composition of acetonitrile from 10 v/v% acetonitrile/water to 20 v/v% acetonitrile/water for 40 min. to obtain 5 mg of lycoricidine represented by chemical formula 1 from 25 g of Lycoris aurea extracts.

NMR analysis and mass analysis were performed as described below to identify the structure of lycoricidine represented by chemical formula 1.

Its molecular weight was determined as 291 through MS measurement using Agilent 1100 high performance liquid chromatography-mass spectrometry (HPLC-ESI-MS), and its structure was identified as chemical formula 1 above through ¹H NMR spectrum analysis using a nuclear magnetic resonance spectroscopy (Varian 500 MHz NMR) (George R. Tettit and Joy A. Bell, J. Nat. Prod 69: 7-13, 2006). The analysis results are as follows.

A light yellow semi-solid substance, molecular formula C₁₄H₁₃NO₆; ESI-MS : m/z 292 [M+H]^{+ 1}H NMR (500 MHz, CD₃OD) : δ 3.92 (1H, m, H-3), 3.94 (1H, m, H-4), 4.26 (1H, ddd, J = 4.5, 2.0, 1.5 Hz, H-2), 4.40 (1H, ddt, J = 9.5, 2.5, 1.0 Hz, H-4a), 6.06 and 6.08 (each 1H, d, J = 1.0 Hz, -OCH₂O-), 6.18 (1H, m, H-1), 7.17 (1H, s, H-10), 7.40 (1H, s, H-7).

### Example 4: Purification and Structure Identification of Lycoricidinol

The inventors concentrated the subfraction (CN1-M-E-fr3) containing the lycoricidinol as a main component prepared in Example 2 under a reduced pressure and then, performed a high performance liquid chromatography separation with a C18 reversed-phase column using a mixture solution of acetonitrile and water with a concentration gradient of increasing the composition of acetonitrile from 10 v/v% acetonitrile/water to 20 v/v% acetonitrile/water for 20 min. to obtain 10 mg of lycoricidinol represented by chemical formula 2 from 25 g of Lycoris aurea extracts.

NMR analysis and mass analysis were performed as described below to identify the structure of lycoricidinol represented by chemical formula 2.

Its molecular weight was determined as 307 through MS measurement using Agilent 1100 high performance liquid chromatography-mass spectrometry (HPLC-ESI-MS), and its structure was identified as chemical formula 2 above through ¹H NMR spectrum analysis using a nuclear magnetic resonance spectroscopy (Varian 500 MHz NMR) (Shanmugham Elango and Yu-Hsin Yan, J. Org. Chem 67: 6954-6959, 2002). The analysis results are as follows.

A light yellow semi-solid substance; molecular formula C₁₄H₁₃NO₇; ESI-MS : m/z 308 [M+H]⁺; ¹H NMR (500 MHz, CD₃OD) : δ 3.91 (1H, m, H-3), 3.92 (1H, m, H-4), 4.24 (1H, m, H-2), 4.36 (1H, m, H-4a), 6.03 and 6.05 (each 1H, d, J = 1.0 Hz, -OCH₂O-), 6.18 (1H, m, H-1), 6.75 (1H, s, H-10).

### Test Example 1: Inhibitory Activity of Lycoris Aurea Extracts against Beta-Amyloid Production

To investigate in vitro inhibitory activity of Lycoris aurea extracts against beta-amyloid production, a HeLa cell line transfected with Human APP was used. This cell line was provided from Professor Tae-Wan Kim (Department of Pathology, Columbia University Medical Center, New York, NY10032, USA). For the quantity analysis of major beta-amyloids, A*β*40, and A*β*42, which are resulted from the activity of gamma-secretase secreted from cells, Human Beta Amyloid [1-40] (A*β*40), and Human Beta Amyloid [1-42] (A*β*42) Colorimetric ELISA Kit (#KHB3482 and #KHB3442; BioSource International, Inc., USA) were each used.

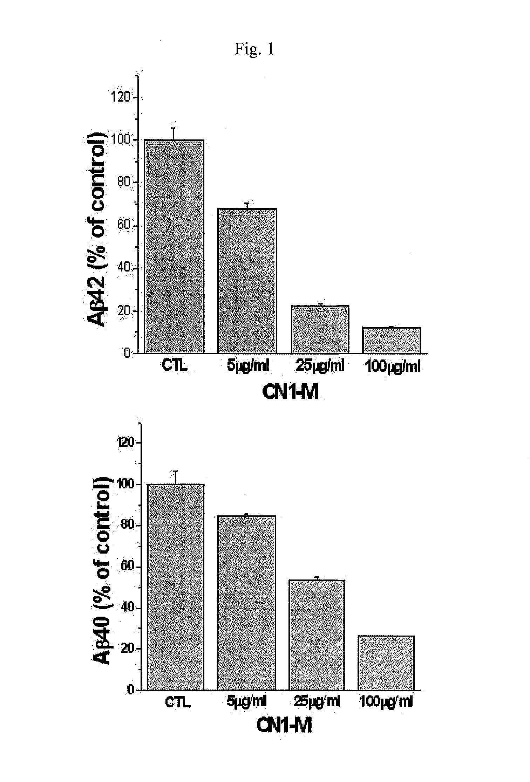

The alcohol extracts of Lycoris aurea (CN1-M, see Example 1) with certain concentrations set forth in Table 1 below were added to the cell culture medium (DMEM culture medium #11995; GIBCO) cultured with the transfected HeLa cells, which were then cultured at 37°C for 8 hours. The quantity of beta-amyloids secreted into the culture medium was measured and shown in Table 1 and Fig. 1. A transfected HeLa cell culture medium with no treatment, to which no Lycoris aurea alcohol extracts were added, was used as a negative control.

**<Table 1> Inhibitory Efficacy on Beta-amyloid Production According to Concentrations of Lycoris Aurea Alcohol Extracts (CN1-M)**

| | 100 *µ*g/ml | 25 *µ*g/ml | 5 *µ*g/ml |
|---|---|---|---|
| A*β*42 Production Inhibitory Rate (%) against Negative Control | 87.8 | 77.5 | 31.9 |
| A*β*40 Production Inhibitory Rate (%) against Negative Control | 73.4 | 46.6 | 15.5 |

As seen in Table 1 and Fig. 1, the alcohol extracts of Lycoris aurea exhibited inhibitory effects on the production of beta-amyloids A*β*42 and A*β*40 in a concentration-dependent manner.

### Test Example 2: Inhibitory Activity by Fractions of Lycoris Aurea Extracts CN1-M against Beta-Amyloid Production

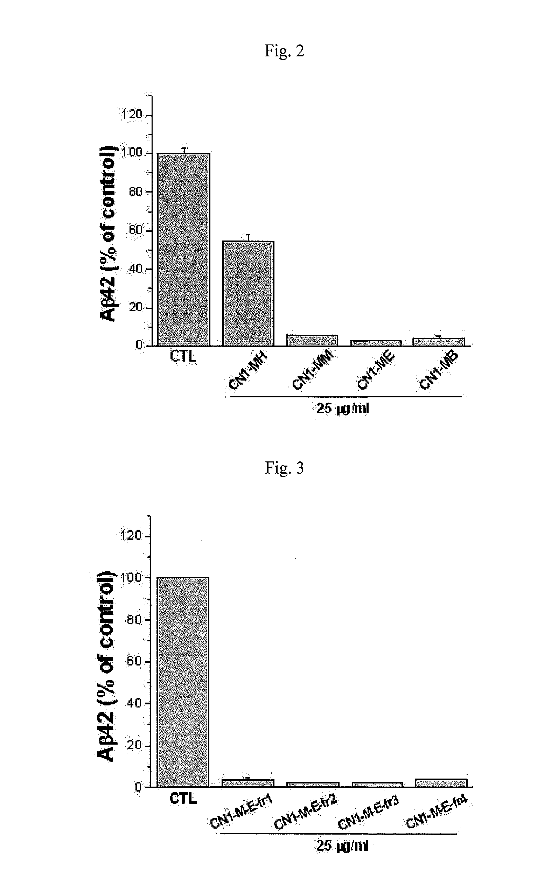

Using the solvent fractions of CN1-M prepared in Example 2, which are a hexane fraction (CN1-MH), methylene chloride fraction (CN1-MM), ethyl acetate fraction (CN1-ME) and butanol fraction (CN1-MB) with a certain concentration (25 *µ*g/ml), the same method as Test Example 1 above was carried out, and the inhibitory activity results against beta-amyloids are shown in Table 2 and Fig. 2. A transfected HeLa cell culture medium with no treatment, to which no solvent fractions were added, was used as a negative control.

**<Table 2> Inhibitory Efficacy of Solvent Fractions (25 µg/ml) of Lycoris Aurea Extracts on Beta-amyloid Production**

| | Hexane Fraction (CN1-MH) | Methylene Chloride Fraction (CN1-MM) | Ethyl Acetate Fraction (CN1-ME) | Butanol Fraction (CN1-MB) |
|---|---|---|---|---|
| A*β*42 Production Inhibitory Rate (%) against Negative Control | 45.3 | 94.4 | 97.0 | 95.7 |

As seen in Table 2 and Fig. 2, of the solvent fractions, the ethyl acetate (CN1-ME) and the butanol fraction (CN1-MB) exhibited excellent inhibitory effects on A*β*42 production.

### Test Example 3: Inhibitory Activity by 4 Types of Subfractions Prepared from the Mixture Fraction (CN1-M-E) of Ethyl Acetate Fraction (CN1-ME) and Butanol Fraction (CN1-MB) against Beta-Amyloid Production

With regard to 4 types of subfractions prepared from the combined fraction (CN1-M-E) of the ethyl acetate fraction and the butanol fraction prepared from Example 2, CN1-M-E-fr1, E-fr2, E-fr3, and E-fr4, their inhibitory activity test on A*β*42 production was carried out by the same method as Test Example 1, and the results are shown in Table 3 and Fig. 3.

**<Table 3> Inhibitory Efficacy by 4 Types of Subfractions (25 µg/ml) Prepared from the Combined Fraction (CN1-M-E) of Ethyl Acetate and Butanol Fractions of Lycoris Aurea CN1-M on Beta-Amyloid Production**

| | fr1 | fr2 | fr3 | fr4 |
|---|---|---|---|---|
| A*β*42 Production Inhibitory Rate (%) against Negative Control | 96.6 | 98.1 | 97.9 | 96.0 |

As seen in Table 3 and Fig. 3, all of the 4 sub fractions exhibited a remarkable inhibitory activity over 96% against beta-amyloid production.

### Test Example 4: Inhibitory Activity by Lycoricidine and Lycoricidinol against Beta-Amyloid Production

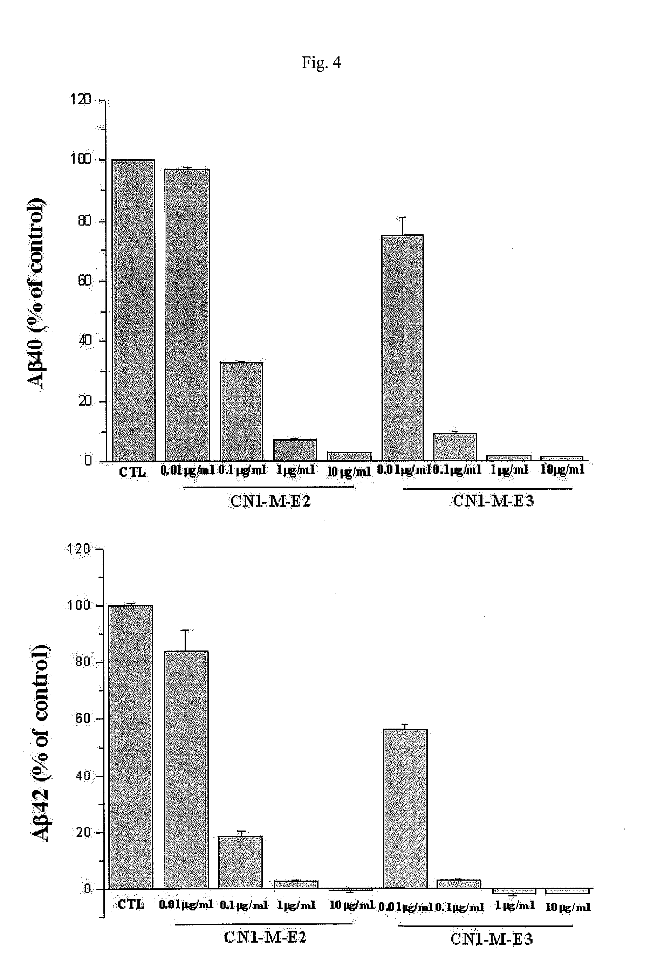

The inhibitory activity test by the compounds, lycoricidine (CN1-M-E2) and lycoricidinol (CN1-M-E3) obtained from Example 3 and Example 4 on A*β*42 and A*β*40 production was carried out by the same method as Test Example 1, and the results are shown in Table 4 and Fig. 4.

**<Table 4> Inhibitory Efficacy of Lycoricidine and Lycoricidinol on Beta-Amyloid Production**

| | Lycoricidine (CN1-M-E2) | | | | Lycoricidinol (CN1-M-E3) | | | |
|---|---|---|---|---|---|---|---|---|
| Concentration (*µ*g/ml) | 0.01 | 0.1 | 1 | 10 | 0.01 | 0.1 | 1 | 10 |
| A*β*42 Production Inhibitory Rate (%) against Negative Control | 16.0 | 81.3 | 97.5 | 100 | 44.0 | 97.0 | 100 | 100 |
| A*β*40 Production Inhibitory Rate (%) against Negative Control | 3.3 | 67.0 | 92.8 | 97.2 | 25.0 | 90.4 | 97.8 | 98.4 |

As seen in Table 4 and Fig. 4, both lycoricidine and lycoricidinol exhibited a remarkable inhibitory activity on beta-amyloid production in a concentration-dependent manner.

### Test Example 5: Alpha-Secretase Activity by Lycoricidine and Lycoricidinol

As proven in Test Example 4, and Table 4 and Fig. 4, the production of A*β*4**2** and A*β*40 was inhibited by lycoricidine and lycoricidinol and as a result, the products of gamma-secretase were decreased. The first possibility to be considered for the cause of a decrease in the production of A*β*42 and A*β*40 is an increase in the activity of alpha-secretase. Also, because the production of A*β*4**2** and A*β*40 is resulted from the successive enzyme activity of beta- and gamma- secretases, there might be a possibility of the suppressive effects by the compounds on beta- and gamma-secretases.

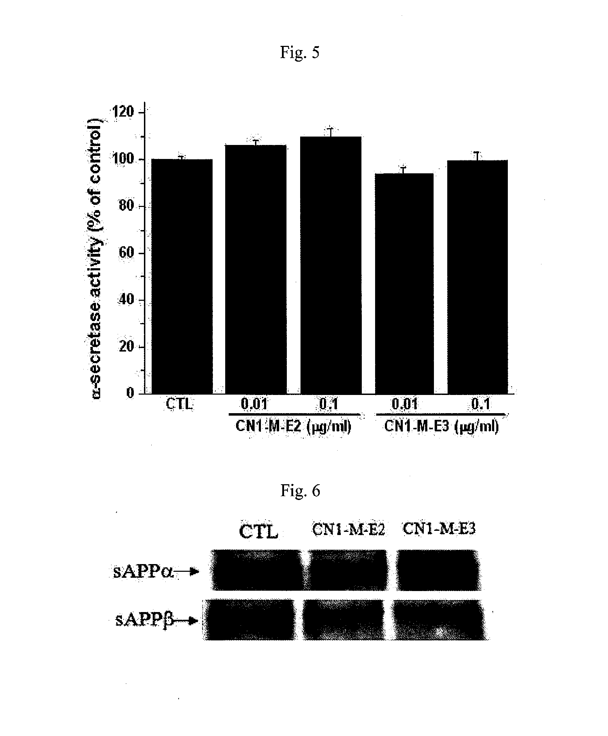

In order to investigate which secretase is responsible for the inhibitory effects of the compounds on A*β* production, an activity assay kit (#CBA042; Calbiochem, USA) of the alpha-secretase was used. For the alpha-secretase, the total membrane proteins obtained from the HeLa cells (see Test Example 1) were used. Any activity changes of alpha-secretase by lycoricidine and lycoricidinol are shown in Table 5 and Fig. 5.

**<Table 5> Activity Change of Alpha-Secretase by Lycoricidine and Lycoricidinol (100%: No Change)**

| | Lycoricidine (CN1-M-E2) | | Lycoricidinol (CN1-M-E3) | |
|---|---|---|---|---|
| Activity Change Rate of Alpha-Secretase (%) against Negative Control | 0.01 *µ*g/ml | 0.1 *µ*g/ml | 0.01 *µ*g/ml | 0.1 *µ*g/ml |
| | 106 | 110 | 94 | 100 |

As seen in Table 5 and Fig. 5, the lycoricidine and lycoricidinol did not change the activity of the alpha-secretase at concentrations of 0.01 and 0.1 *µ*g/ml, which were the concentrations at which the compounds effectively inhibited the production of A*β*42 and A*β*40 in Test Example 4 (see Table 4 and Fig. 4). Therefore, it can be concluded that the inhibition by lycoricidine and lycoricidinol against the production of A*β*42 and A*β*40 is not caused by the activity change of the alpha-secretase.

### Test Example 6: Inhibitory Activity against Production of Products of Alpha-, or Beta-Secretases by Compounds, Lycoricidine and Lycoricidinol, Prepared from Lycoris Aurea Extracts

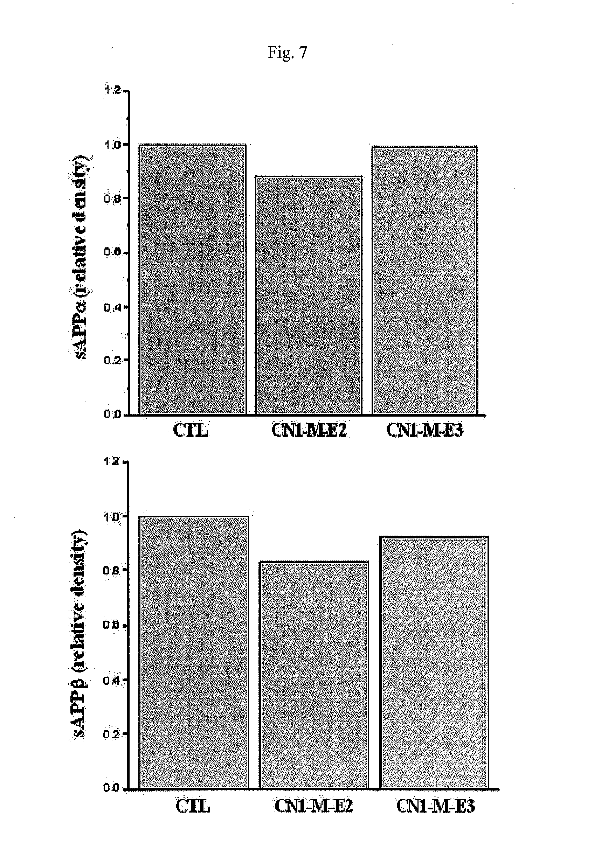

Effects of the compounds on the production of a product resulted from the activity of alpha-secretase, sAPP*α*, and a product resulted from the activity of beta-secretase, sAPP*β*, were investigated through the Western blot methods of sAPPα and sAPP*β*, and the results are shown in Fig. 6. As seen in Fig. 6, the lycoricidine and the lycoricidinol did not affect the secretion of both sAPPα and sAPP*β* at the concentration of 0.1 *µ*g/ml which has an effect on the production of A*β*42 and A*β*40. The band densities of sAPPα and sAPP*β* were measured from the Western blot results of Fig. 6, and the results are shown Table 6 and Fig. 7.

**<Table 6> Production Change of sAPPα and sAPPβ by Lycoricidine and Lycoricidinol**

| | Lycoricidine (CN1-M-E2) | Lycoricidinol (CN1-M-E3) |
|---|---|---|
| Reduction Rate of sAPPα (%) Against Negative Control | 12 | 1.0 |
| Reduction Rate of sAPP*β* (%) Against Negative Control | 17 | 7.5 |

As seen in Table 6 and Fig. 7, the production changes of sAPPα and sAPP*β* by lycoricidine and lycoricidinol are not significant. Therefore, it can be concluded that the reduction effects on the production of A*β*42 and A*β*40 by lycoricidine and lycoricidinol are not resulted from changes in the activity of alpha-, and beta-secretases.

To put these results together, it can be concluded that the production inhibition of A*β*4**2** and A*β*40 by lycoricidine and lycoricidinol are resulted from the suppression of the activity of gamma-secretase by these compounds.

### Test Example 7: Cell Death Activity by Compounds Lycoricidine and Lycoricidinol

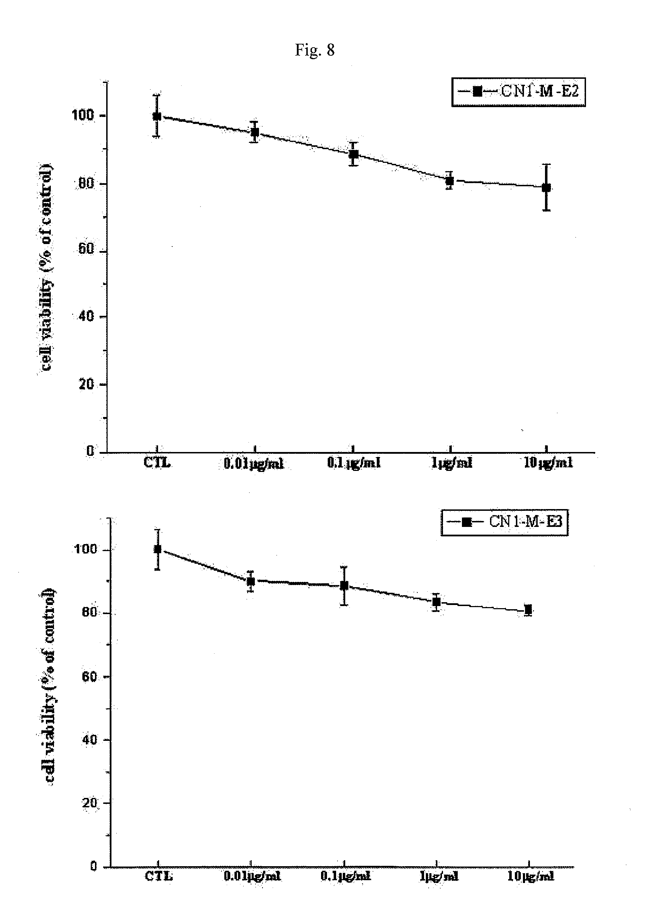

In order to investigate the cell death activity caused by the compounds, lycoricidine and lycoricidinol, MTT Cell Proliferation assay method was used (ATCC catalog #30-1010K, Manassas, USA). After cells were treated with each compound having various concentrations for 8 hours, viable cells were measured quantitatively, and the results are shown in Table 7 and Fig. 8.

**<Table 7> Cell Death by Lycoricidine and Lycoricidinol**

| | Lycoricidine (CN1-M-E2) | | | | Lycoricidinol (CN1-M-E3) | | | |
|---|---|---|---|---|---|---|---|---|
| Concentration (*µ*g/ml) | 0.01 | 0.1 | 1 | 10 | 0.01 | 0.1 | 1 | 10 |
| Cell Death Rate (%) against Negative Control | 4.9 | 11.3 | 19 | 21.1 | 10.3 | 11.6 | 16.5 | 19.3 |

As seen in Table 7 and Fig. 8, even when 10 *µ*g/ml of lycoricidine and lycoricidinol which was the highest concentration used in the experiments was treated, only cells within about 20% died. From such results, it can be concluded that the reduction effects on the production of beta-amyloid by the compounds are not resulted from simple cell death and the compounds are not cytotoxic.

## Claims

1. A composition for prevention or treatment of dementia containing Lycoris aurea extract as an active ingredient.

2. A composition for inhibition of beta-amyloid containing Lycoris aurea extract as an active ingredient.

3. The composition of Claim 1 or 2, wherein the Lycoris aurea extract is obtained by extracting Lycoris aurea using one or more solvents selected from the group consisting of water and a short-chain alcohol of C1 to C4.

4. The composition of Claim 1 or 2, wherein the Lycoris aurea extract is an ethyl acetate fraction, a butanol fraction, or a mixture thereof, wherein the fractions are obtained by extracting Lycoris aurea using one or more solvents selected from the group consisting of water and a short-chain alcohol of C1 to C4 and fractioning the obtained extract using hexane, methylene chloride, ethyl acetate, and butanol in sequence.

5. The composition of Claim 1 or 2, wherein the Lycoris aurea extract is a subfraction obtained by extracting Lycoris aurea using one or more solvents selected from the group consisting of water and a short-chain alcohol of C1 to C4, fractioning the obtained extract using hexane, methylene chloride, ethyl acetate and butanol in sequence to obtain solvent fractions, and performing a reversed-phase column chromatography for a mixture of an ethyl acetate fraction and a butanol fraction among the solvent fractions, using a mixture of acetonitrile and water as an eluent.

6. The composition of Claim 5, wherein the reversed-phase column chromatography is performed with increasing the content of acetonitrile in the mixture of acetonitrile and water from 10 v/v% to 50 v/v% for 60 min.

7. The composition of Claim 6, wherein the content of acetonitrile in the mixture of acetonitrile and water is 31 to 33 v/v%, and the obtained subfraction contains lycoricidine represented by chemical formula 1 below in an amount of 80 to 99.9 w/w%.

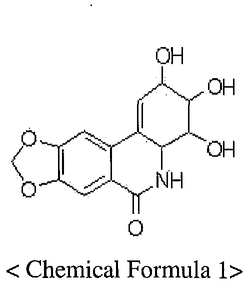

8. The composition of Claim 6, wherein the content of acetonitrile in the mixture solution of acetonitrile and water is 42 to 44 v/v%, and the obtained subfraction contains lycoricidinol represented by chemical formula 2 below in an amount of 80 to 99.9 w/w%.

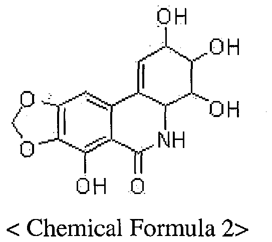

9. A composition for prevention or treatment of dementia containing at least one selected from the group consisting of lycoricidine represented by chemical formula 1 below, lycoricidinol represented by chemical formula 2 below, and a pharmaceutically acceptable salt thereof, as an active ingredient.

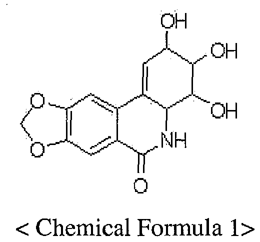

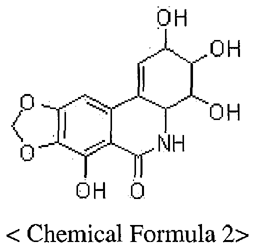

10. A composition for inhibition of beta-amyloid containing at least one selected from the group consisting of lycoricidine represented by chemical formula 1 below, lycoricidinol represented by chemical formula 2 below, and a pharmaceutically acceptable salt thereof, as an active ingredient.

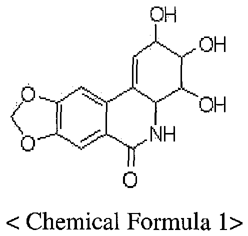

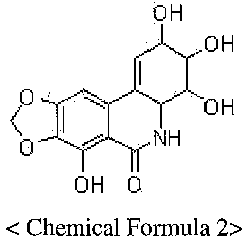

11. The composition for prevention or treatment of dementia according to Claim 1 or Claim 9, wherein the dementia is Alzheimer's disease.

12. A health functional food for prevention or improvement of dementia, comprising the composition for prevention or treatment of dementia according to any one of Claims 1 to Claims 11.

13. A method for preparing Lycoris aurea extract having an inhibitory effect against beta-amyloid production, comprising
extracting Lycoris aurea using one or more solvents selected from the group consisting of water and a short-chain alcohol of C1 to C4;
adding hexane, methylene chloride, ethyl acetate and butanol to the obtained extract in sequence to obtain solvent fractions; and
mixing an ethyl acetate fraction and a butanol fraction among the obtained solvent fractions and then performing a reversed-phase column chromatography using a mixture of acetonitrile and water as an eluent to obtain a subfraction.

14. The method for preparation of Claim 13, wherein the reversed-phase column chromatography is performed with increasing the content of acetonitrile in the mixture of acetonitrile and water from 10 v/v% to 50 v/v% for 60 min.

15. The method for preparation of Claim 14, further comprising obtaining a subfraction from the eluted solution when the content of acetonitrile in the mixture of acetonitrile and water is 31 to 33 v/v%,
wherein the obtained subfraction contains lycoricidine represented by chemical formula 1 below in an amount of 80 to 99.9 w/w%:

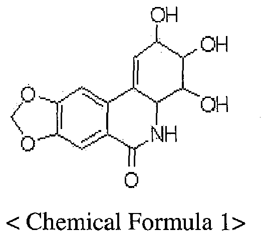

16. The method for preparation of Claim 14, further comprising obtaining a subfraction from the eluted solution when the content of acetonitrile in the mixture of acetonitrile and water is 42 to 44 v/v%,
wherein the obtained subfraction contains lycoricidinol represented by chemical formula 2 below in an amount of 80 to 99.9 w/w%:

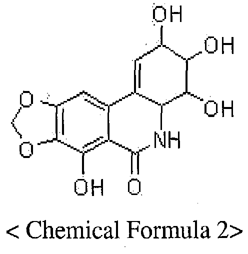

17. A use of Lycoris aurea extract for preparation of a medicine for dementia.

18. A use of Lycoris aurea extract for preparation of an inhibitor of beta-amyloid.

19. The use of Claim 17 or Claim 18, wherein the Lycoris aurea extract is obtained by extracting Lycoris aurea using one or more solvents selected from the group consisting of water and a short-chain alcohol of C 1 to C4.

20. The use of Claim 17 or 18, wherein the Lycoris aurea extract is an ethyl acetate fraction, a butanol fraction, or a mixture thereof, wherein the fractions are obtained by extracting Lycoris aurea using one or more solvents selected from the group consisting of water and a short-chain alcohol of C1 to C4 and fractioning the obtained extract using hexane, methylene chloride, ethyl acetate and butanol in sequence.

21. The use of Claim 17 or 18, wherein the Lycoris aurea extract is a subfraction obtained by extracting Lycoris aurea using one or more solvents selected from the group consisting of water and a short-chain alcohol of C1 to C4, fractioning the obtained extract using hexane, methylene chloride, ethyl acetate and butanol in sequence to obtain solvent fractions, and performing a reversed-phase column chromatography for a mixture of an ethyl acetate fraction and a butanol fraction among the obtained solvent fractions, using a mixture of acetonitrile and water as an eluent.

22. The use of Claim 21, wherein the reversed-phase column chromatography is performed with increasing the content of acetonitrile in the mixture of acetonitrile and water from 10 v/v% to 50 v/v% for 60 min.

23. The use of Claim 22, wherein the content of acetonitrile in the mixture of acetonitrile and water is 31 to 33 v/v%, and the obtained subfraction contains lycoricidine represented by chemical formula 1 below in an amount of 80 to 99.9 w/w%:

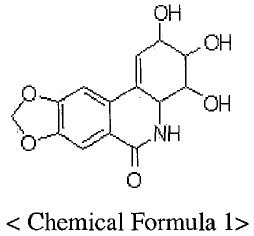

24. The use of Claim 22, wherein the content of acetonitrile in the mixture of acetonitrile and water is 42 to 44 v/v%, and the obtained subfraction contains lycoricidinol represented by chemical formula 2 below in an amount of 80 to 99.9 w/w%:

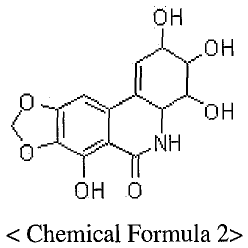

25. A use of at least one selected from the group consisting of lycoricidine represented by chemical formula 1 below, lycoricidinol represented by chemical formula 2 below, and a pharmaceutically acceptable salt thereof, for preparation of a medicine for dementia.

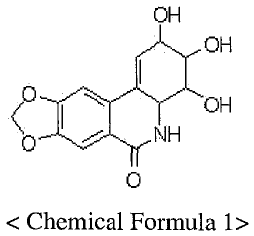

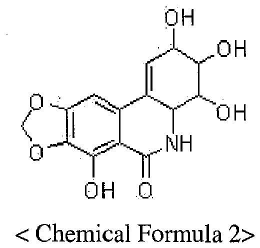

26. A use of at least one selected from the group consisting of lycoricidine represented by chemical formula 1 below, lycoricidinol represented by chemical formula 2 below, and a pharmaceutically acceptable salt thereof, for preparation of an inhibitor of beta-amyloid.

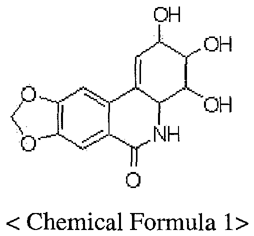

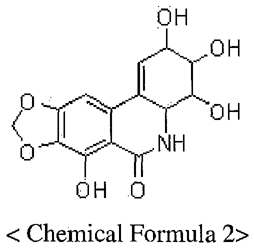

27. The use of Claim 1 or Claim 25, wherein the dementia is Alzheimer's disease.

28. A method of preventing or treating dementia comprising administering Lycoris aurea extract to a patient in need of preventing or treating dementia.

29. The method of Claim 28, wherein the Lycoris aurea extract is obtained by extracting Lycoris aurea using one or more solvents selected from the group consisting of water and a short-chain alcohol of C1 to C4.

30. The method of Claim 28, wherein the Lycoris aurea extract is an ethyl acetate fraction, a butanol fraction, or a mixture thereof, wherein the fractions are obtained by extracting Lycoris aurea using one or more solvents selected from the group consisting of water and a short-chain alcohol of C1 to C4 and fractioning the obtained extract using hexane, methylene chloride, ethyl acetate and butanol in sequence.

31. The method of Claim 28, wherein the Lycoris aurea extract is a subfraction obtained by extracting Lycoris aurea using one or more solvents selected from the group consisting of water and a short-chain alcohol of C1 to C4, fractioning the obtained extract using hexane, methylene chloride, ethyl acetate and butanol in sequence to obtain solvent fractions, and performing a reversed-phase column chromatography for a mixture of an ethyl acetate fraction and a butanol fraction among the solvent fractions, using a mixture of acetonitrile and water as an eluent.

32. The method of Claim 28, wherein the reversed-phase column chromatography is performed with increasing the content of acetonitrile in the mixture of acetonitrile and water from 10 v/v% to 50 v/v% for 60 min.

33. The method of Claim 32, wherein the content of acetonitrile in the mixture of acetonitrile and water is 31 to 33 v/v%, and the obtained subfraction contains lycoricidine represented by chemical formula 1 below in an amount of 80 to 99.9 w/w%:

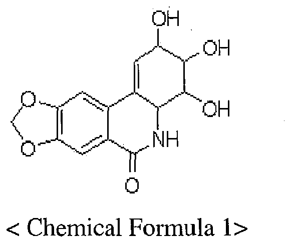

34. The method of Claim 32, wherein the content of acetonitrile in the mixture of acetonitrile and water is 42 to 44 v/v%, and the obtained subfraction contains lycoricidinol represented by chemical formula 2 below in an amount of 80 to 99.9 w/w%:

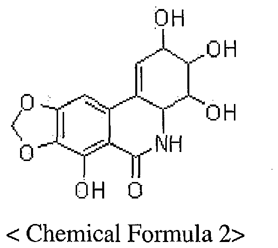

35. A method of preventing or treating dementia comprising administering at least one selected from the group consisting of lycoricidine represented by chemical formula 1 below, lycoricidinol represented by chemical formula 2 below, and a pharmaceutically acceptable salt thereof, to a patient in need of preventing or treating dementia.

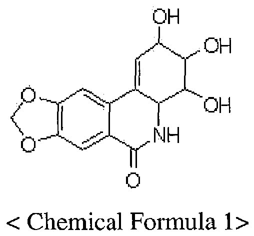

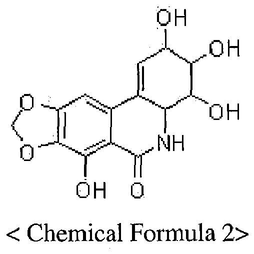

36. The method of Claim 28, wherein the dementia is Alzheimer's disease.

37. A method of inhibiting beta-amyloid comprising administering Lycoris aurea extract to a patient in need of inhibiting beta-amyloid.

38. The method of Claim 37, wherein the Lycoris aurea extract is obtained by extracting Lycoris aurea using one or more solvents selected from the group consisting of water and a short-chain alcohol of C1 to C4.

39. The method of Claim 37, wherein the Lycoris aurea extract is an ethyl acetate fraction, a butanol fraction, or a mixture thereof, wherein the fractions are obtained by extracting Lycoris aurea using one or more solvents selected from the group consisting of water and a short-chain alcohol of C1 to C4 and fractioning the obtained extract using hexane, methylene chloride, ethyl acetate and butanol in sequence.

40. The method of Claim 37, wherein the Lycoris aurea extract is a subfraction obtained by extracting Lycoris aurea using one or more solvents selected from the group consisting of water and a short-chain alcohol of C 1 to C4, fractioning the obtained extract using hexane, methylene chloride, ethyl acetate and butanol in sequence to obtain solvent fractions, and performing a reversed-phase column chromatography for a mixture of an ethyl acetate fraction and a butanol fraction among the solvent fractions, using a mixture of acetonitrile and water as an eluent.

41. The method of Claim 37, wherein the reversed-phase column chromatography is performed with increasing the content of acetonitrile in the mixture of acetonitrile and water from 10 v/v% to 50 v/v% for 60 min.

42. The method of Claim 41, wherein the content of acetonitrile in the mixture of acetonitrile and water is 31 to 33 v/v%, and the obtained subfraction contains lycoricidine represented by chemical formula 1 below in an amount of 80 to 99.9 w/w%:

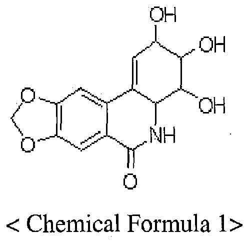

43. The method of Claim 41, wherein the content of acetonitrile in the mixture of acetonitrile and water is 42 to 44 v/v%, and the obtained subfraction contains lycoricidinol represented by chemical formula 2 below in an amount of 80 to 99.9 w/w%:

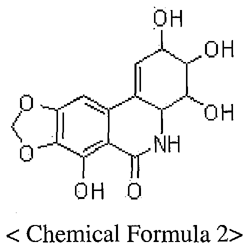

44. A method of inhibiting beta-amyloid comprising administering at least one selected from the group consisting of lycoricidine represented by chemical formula 1 below, lycoricidinol represented by chemical formula 2 below, and a pharmaceutically acceptable salt thereof, to a patient in need of inhibiting beta-amyloid.
